# EUROPÄISCHE PATENTSCHRIFT

(11) **EP 0 832 878 B1**
(45) Veröffentlichungstag und Bekanntmachung des Hinweises auf die Patenterteilung: **11.09.2002**
(21) Anmeldenummer: 97111738.7
(22) Anmeldetag: 10.07.1997
(51) Int. Cl.: C07C 319/08, B01J 23/04, B01J 23/30

(54) **Katalysator, Verfahren zu seiner Herstellung und Verwendung zur Synthese von Methylmercaptan**
Catalyst, process for its production and use in the synthesis of methylmercaptan
Catalyseur, son procédé de préparation et son utilisation pour la synthèse de méthylmercaptan

(30) Priorität: 26.09.1996 DE 19639520
(43) Veröffentlichungstag der Anmeldung: 01.04.1998
(73) Patentinhaber: Degussa AG, 40474 Düsseldorf (DE)
(72) Erfinder: Sauer, Jörg, Dr., 63517 Rodenbach (DE); von Hippel, Lukas, Dr., 63755 Alzenau (DE); Arntz, Dietrich, Dr., 63829 Oberursel (DE); Böck, Wolfgang, Dr., 63505 Langenselbold (DE)

(56) Entgegenhaltungen:
- US-A- 2 820 062
- A. V. MASHKINA ET AL.: "Activity of tungstate catalysts in the synthesis of methylmercaptane from methanol and hydrogen sulfide" REACT. KINET. CATAL. LETT., Bd. 36, Nr. 1, 1988, Seiten 159-164, XP002063476

## Beschreibung

Die vorliegende Erfindung betrifft einen Katalysator zur Synthese von Methylmercaptan aus Methanol und Schwefelwasserstoff, sowie ein Verfahren zur Herstellung dieses Katalysators.

Methylmercaptan ist ein industriell wichtiges Zwischenprodukt für die Synthese von Methionin sowie für die Herstellung von Dimethylsulfoxid und Dimethylsulfon. Es wird heute überwiegend aus Methanol und Schwefelwasserstoff durch Reaktion an einem Katalysator aus Aluminiumoxid hergestellt. Die Synthese des Methylmercaptans erfolgt gewöhnlich in der Gasphase bei Temperaturen zwischen 300 und 500°C und bei Drucken zwischen 1 und 25 bar.

Das Reaktionsgasgemisch enthält neben dem gebildeten Methylmercaptan die nicht umgesetzten Ausgangsstoffe und Nebenprodukte, wie zum Beispiel Dimethylsulfid und Dimethyläther sowie die im Sinne der Reaktion inerten Gase, wie zum Beispiel Methan, Kohlenoxid, Wasserstoff und Stickstoff. Aus diesem Reaktionsgemisch wird das gebildete Methylmercaptan abgetrennt.

Wird die Reaktion von Schwefelwasserstoff und Methanol am Katalysator bei erhöhtem Druck durchgeführt und fällt somit das Produkt der Methylmercaptan-Herstellung bei erhöhtem Druck (mehr als 7 bar) an, kann, wie in DE-B-17 68 826 beschrieben ist, Methylmercaptan beispielsweise durch Wäsche mit Methanol bei einer Temperatur am Kopf des Wäschers von 25°C abgetrennt werden. Fällt das Reaktionsprodukt bei Normaldruck an, muß in der Aufarbeitung bei Temperaturen bis -60°C gearbeitet werden (JP-B- 45-10728), um das Methylmercaptan in flüssiger Form gewinnen zu können. Der nicht umgesetzte Schwefelwasserstoff kann, wie in DE-B-17 68 826 beschrieben, wieder zum Reaktor zurückgeführt werden.

Für die Wirtschaftlichkeit des Verfahrens wird eine möglichst hohe Selektivität bei der katalytischen Reaktion von Methanol und Schwefelwasserstoff zu Methylmercaptan gefordert, um den Aufwand bei der Abtrennung des gebildeten Methylmercaptans vom Reaktionsgasgemisch so gering wie möglich zu halten. Hier stellt insbesondere der Energieaufwand zur Kühlung des Reaktionsgasgemisches zur Auskondensation des Methylmercaptans einen großen Kostenfaktor dar.

Zur Erhöhung von Aktivität und Selektivität des Aluminiumoxidkatalysators wird dieser üblicherweise mit Kaliumwolframat promotiert. Dabei wird der Promotor gewöhnlich in Mengen bis 15 Gew.-%, bezogen auf das Gesamtgewicht des Katalysators, eingesetzt. Eine Verbesserung von Aktivität und Selektivität erhält man auch durch Erhöhung des Molverhältnisses von Schwefelwasserstoff zu Methanol. Üblicherweise werden Molverhältnisse zwischen 1 und 10 angewendet.

Ein hohes Molverhältnis bedeutet allerdings auch einen hohen Überschuß des Schwefelwasserstoffes im Reaktionsgasgemisch und somit die Notwendigkeit, große Gasmengen im Kreis zu führen. Zur Verminderung des hierfür benötigten Energieaufwandes sollte daher das Verhältnis von Schwefelwasserstoff zu Methanol nur wenig von 1 abweichen. Weiterhin ist es zur Verminderung der Wärmeverluste am Reaktor wünschenswert, die Reaktion bei möglichst geringen Temperaturen durchzuführen.

Die US-PS 2,820,062 beschreibt ein Verfahren zur Herstellung von organischen Thiolen, welches einen Katalysator aus aktivem Aluminiumoxid verwendet, der mit Kaliumwolframat in einer Menge von 1,5 bis 15 Gew.-%, bezogen auf das Gewicht des Katalysators, promotiert ist. Mit diesem Katalysator werden gute Aktivitäten und Selektivitäten bei Reaktionstemperaturen von 400°C und Molverhältnissen von 2 erzielt. Diese US-Patentschrift nennt verschiedene Möglichkeiten zur Einbringung des Kaliumwolframats in das Aluminiumoxid. So sollen Imprägnierverfahren, Copräzipitationen und reine Mischungen anwendbar sein. Der eigentlichen Herstellung des Katalysators wird wenig Bedeutung für die Wirtschaftlichkeit des Syntheseverfahrens von Methylmercaptan eingeräumt.

Aufgabe der vorliegenden Erfindung ist es, einen Katalysator und ein Verfahren zu seiner Herstellung anzugeben, welcher sich bei niedrigen Molverhältnissen von Schwefelwasserstoff zu Methanol durch verbesserte Aktivität und Selektivität gegenüber den bekannten Katalysatoren auszeichnet und somit zu einer besseren Wirtschaftlichkeit des Verfahrens führt.

Diese Aufgabe wird durch einen Katalysator aus Aluminiumoxid gelöst, der auf aktivem Aluminiumoxid 5 bis 25 Gew.-% Kaliumwolframat als Promotor enthält. Dieser Katalysator ist dadurch erhältlich, daß der Promotor in zwei Portionen auf dem aktiven Aluminiumoxid abgeschieden wird, wobei das Aluminiumoxid zunächst mit der ersten Portion des Promotors durch Imprägnieren in einem Überschuß einer wäßrigen Imprägnierlösung beschichtet und danach bei erhöhter Temperatur getrocknet wird, bevor die zweite Portion mittels der Porenvolumenimprägnierung auf dem Aluminiumoxid aufgebracht und die so erhaltene Katalysatorvorstufe nach erneutem Trocknen bei erhöhter Temperatur abschließend bei Temperaturen zwischen 200 und 600°C calciniert wird.

Das Mengenverhältnis der beiden Portionen des Promotors wird bevorzugt so eingestellt, daß die Menge der ersten Portion des Promotors ein Drittel bis zwei Drittel der Gesamtmenge des Promotors ausmacht. Als Promotoren können Wolframate der Alkalielemente Li, Na, K und Rb oder Mischungen davon verwendet werden, bevorzugt wird jedoch nur Kalium eingesetzt.

Als Aluminiumoxid für diesen Katalysator wird sogenanntes aktives Aluminiumoxid eingesetzt. Dieses Material weist hohe spezifische Oberflächen zwischen etwa 10 und 400 m²/g auf und besteht hauptsächlich aus Oxiden der Übergangsreihe der kristallographischen Phasen des Aluminiumoxids (siehe zum Beispiel Ullmann's Enzyclopedia of Industrial Chemistry von 1985, Vol. A1, Seiten 561 - 562). Zu diesen Übergangsoxiden gehören χ-, κ-, γ-, δ-, η- und θ-Aluminiumoxid. Alle diese kristallographischen Phasen gehen bei Erhitzung des Aluminiumoxids auf Temperaturen über 1100°C in das thermisch stabile α-Aluminiumoxid über. Aktives Aluminiumoxid wird kommerziell für katalytische Anwendungen in verschiedenen Qualitäten und Lieferformen angeboten. Gut geeignet für die Zwecke der Erfindung ist granuliertes oder stranggepreßtes Aluminiumoxid mit Korndurchmessern von 1 bis 5 mm, einer spezifischen Oberfläche von 180 - 400 m²/g, einem Gesamtporenvolumen zwischen 0,3 und 1,0 ml/g sowie einer Schüttdichte von 300 bis 900 g/l.

Der Promotor Kaliumwolframat wird durch eine zweistufige Imprägnierung auf dem Aluminiumoxid abgeschieden, wobei das Aluminiumoxid in der ersten Stufe in einem Überschuß der wäßrigen Imprägnierlösung mit dem oder den Promotoren imprägniert und in der zweiten Stufe durch Porenvolumenimprägnierung mit der restlichen Menge Promotor beschichtet wird. Diese spezielle zweistufige Imprägnierung mit Zwischentrocknung führt gegenüber der aus dem Stand der Technik bekannten einstufigen Imprägnierung zu einer erhöhten Aktivität und Selektivität des fertigen Katalysators insbesondere bei niedrigen Molverhältnissen von Schwefelwasserstoff zu Methanol.

Die Art der beiden Imprägnierschritte ist von entscheidender Bedeutung für die Aktivität und Selektivität des fertigen Katalysators.

Zur Durchführung des ersten Imprägnierschrittes wird eine wäßrige Lösung des Promotors hergestellt. Falls die gewünschte Konzentration und/oder die begrenzte Löslichkeit der Promotorverbindung es erfordern, kann die Temperatur der Imprägnierlösung bis auf 95°C angehoben werden. Die in einem Gefäß vorgelegten Katalysatorpartikel aus Aluminiumoxid werden dann mit der gegebenenfalls noch warmen Imprägnierlösung übergossen, bis alle Partikel von der Lösung bedeckt sind. Nach Abscheidung der Promotorverbindung, was etwa nach 20 bis 60 Minuten der Fall ist, wird das überschüssige Wasser abgegossen.

Nach dem Abgießen des Wassers werden die Katalysatorpartikel während einer Dauer von 1 bis 10 Stunden bei erhöhter Temperatur zwischen 50 und 250°C, bevorzugt zwischen 100 und 140°C, getrocknet. Danach kann sich eine Calcinierbehandlung bei Temperaturen zwischen 400 und 600°C, insbesondere zwischen 420 und 480°C, für die Dauer von 1 bis 5 Stunden anschließen. Der Trocknung kann eine Vortrocknung bei Raumtemperatur für die Dauer bis zu 20, bevorzugt 10 bis 14 Stunden, vorangehen. Dadurch wird die Gleichmäßigkeit der Imprägnierung über den Querschnitt der Katalysatorpartikel verbessert.

Im zweiten Imprägnierschritt wird die restliche Menge des Promotors durch Porenvolumenimprägnierung (auch als "incipient-wetness-impregnation" bekannt) in die Katalysatorträger eingebracht. Hierzu wird die restliche Menge des Promotors in einem Volumen Wasser gelöst, welches etwa dem Wasseraufnahmevermögen der Katalysatorpartikel entspricht. Hierbei kann die Lösung wieder zur Verbesserung der Löslichkeit bis auf 95°C erwärmt werden. Diese Lösung wird langsam über die in einem Dragierkessel umgewälzten Katalysatorträger verteilt. Danach werden die Katalysatorträger wie nach der ersten Imprägnierung getrocknet und anschließend bei Temperaturen zwischen 400 und 600°C für die Dauer von 1 bis 5 Stunden calciniert.

Vor dem Aufbringen des Promotors empfiehlt es sich, die Katalysatorkörper ebenfalls bei Temperaturen zwischen 400 und 600°C für die Dauer von 1 bis 10 Stunden vorzucalcinieren, um optimale Aktivitätswerte zu erzielen.

Die in den beiden Imprägnierstufen aufgebrachten Mengen des Promotors werden bevorzugt gleich groß gewählt. Die Menge des in der ersten Imprägnierstufe aufgebrachten Promotors kann jedoch auch zwischen einem Drittel und zwei Drittel der Gesamtmenge des Promotors variieren.

Dem beschriebenen Ablauf der Imprägnierschritte kommt eine entscheidende Bedeutung für die Ausbildung einer hohen Aktivität und Selektivität der fertigen Katalysatoren zu. Vergleichsversuche mit einstufigen Imprägnierungen im Überschuß und mit ein- und zweistufigen Porenvolumenimprägnierungen führten zu Katalysatoren mit geringerer Aktivität und Selektivität.

Vor dem Einsatz bei der Methylmercaptansynthese werden die Katalysatoren unter reaktionsähnlichen Bedingungen vorsulfidiert ((V.Yu. Mashkin, Appl. Catal. A 109 (1994) S. 45 - 61). Hierzu wird ein Schwefelwasserstoffstrom bei 350°C und einem Druck von 9 bar für die Dauer von 2 Stunden über die Katalysatorpartikel geleitet. Während erfindungsgemäße und konventionelle Katalysatoren vor dieser Behandlung äußerlich grau gefärbt sind und einen weißen Kern besitzen, werden nach der Sulfidierung deutliche Farbunterschiede beobachtet. Konventionell hergestellte Katalysatoren zeigen nur geringe Farbänderungen nach der Sulfidierung, während die erfindungsgemäß hergestellten Katalysatoren nach der Sulfidierung eine deutliche Gelbfärbung aufweisen, welche auf eine verstärkte Bildung von Thiowolframaten zurückzuführen ist. Diese Gelbfärbung ist nicht nur oberflächlich, sondern durchdringt die Katalysatorpartikel vollständig.

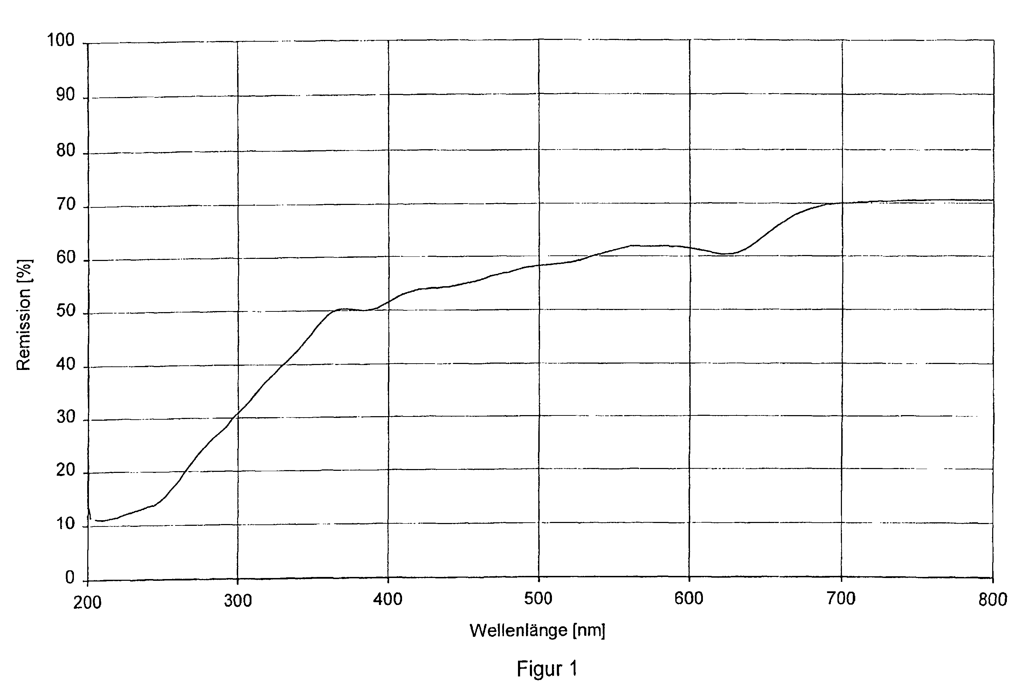

Die beobachtete Gelbfärbung äußert sich im Remissionsspektrum von zu Pulver vermahlenen Katalysatorpartikeln gegenüber konventionellen Katalysatoren durch ausgeprägte Absorptionsbanden zwischen 270 und 420 nm.
Besonders ausgeprägt ist eine Absorptionsbande im Wellenlängenbereich zwischen 375 und 420 nm. Etwas schwächere Absorptionsbanden befinden sich in den Wellenlängenbereichen zwischen 270 und 290 nm und zwischen 325 und 345 nm. Die Remissionsspektren erfindungsgemäßer Katalysatoren sowie von Vergleichskatalysatoren zeigen die Figuren 1 bis 3.
- Figur 1:: Remissionsspektrum des Katalysators von Vergleichsbeispiel 1 gemessen gegen einen Bariumsulfat-Weißstandard.
- Figur 2:: Remissionsspektrum des Katalysators von Beispiel 1 gemessen gegen einen Bariumsulfatweißstandard
- Figur 3:: Remissionsspektrum des Katalysators von Beispiel 1 gemessen gegen den Katalysator von Vergleichsbeispiel 1.

### Beispiel 1

1,8 Kg Aluminiumoxidgranulat (Spheralite 501 A von Rhône-Poulenc; spezifische Oberfläche 320 m²/g; totales Porenvolumen 0,45 ml/g; Schüttdichte 0,8 g/cm³) wurden 4 Stunden lang bei 455°C an Luft calciniert. Über das in einem Gefäß befindliche Granulat wurde eine vorbereitete Lösung von 8,7 Gew.-% Kaliumwolframat in Wasser mit einer Temperatur von 95°C gegossen, bis alle Katalysatorpartikel bedeckt waren. Nach einer Wartezeit von 40 Minuten wurde das überschüssige Wasser abgegossen, die feuchten Katalysatorteilchen 16 Stunden an Luft bei Raumtemperatur vorgetrocknet und anschließend 2 Stunden bei 120°C getrocknet. Auf den Katalysatorpartikeln hatten sich durch diese Behandlung 7 Gew.-% Kaliumwolframat, d. h. 126 g, abgelagert.

Zur Durchführung der Porenvolumenimprägnierung wurden 162 g Kaliumwolframat in 900 ml Wasser, entsprechend 100 % des gemessenen, totalen Porenvolumens des Katalysatormaterials, bei einer Temperatur von 95°C gelöst und über die in einem Dragierkessel umgewälzten Katalysatorträger verteilt. Daran schloß sich wieder eine 16-stündige Vortrocknung an Luft an, gefolgt von einer zweistündigen Trocknung bei 110°C. Abschließend wurden die Katalysatorteilchen 4 Stunden bei 455°C an Luft calciniert.

Der fertige Katalysator enthielt somit 288 g Kaliumwolframat auf 1,8 Kg Aluminiumoxid, also 16 Gew.-% Kaliumwolframat bezogen auf das Gewicht des eingesetzten Katalysatormaterials.

### Beispiel 2

Es wurde wie in Beispiel 1 ein erfindungsgemäßer Katalysator mit 16 Gew.-% Kaliumwolframat hergestellt. Im Unterschied zu Beispiel 1 wurde das Katalysatormaterial vor dem Aufbringen des Promotors nicht calciniert.

### Vergleichsbeispiel 1

Wie in dem vorangegangenen Beispiel 1 wurde ein Katalysator mit 16 Gew.-% Kaliumwolframat hergestellt.Dabei wurde von 1,5 Kg Aluminiumoxid ausgegangen.

Es wurde eine heiße Lösung (95°C) von 17,4 Gew.-% Kaliumwolframat in Wasser angefertigt und damit die vorgelegten Katalysatorpartikel übergossen, bis sie vollständig mit Lösung bedeckt waren. Nach 40 Minuten wurde das überschüssige Wasser abgegossen und das Katalysatormaterial wie in Beispiel 1 vorgetrocknet, getrocknet und calciniert. Der fertige Katalysator enthielt 16 Gew.-% Kaliumwolframat bezogen auf das Gewicht des Aluminiumoxids.

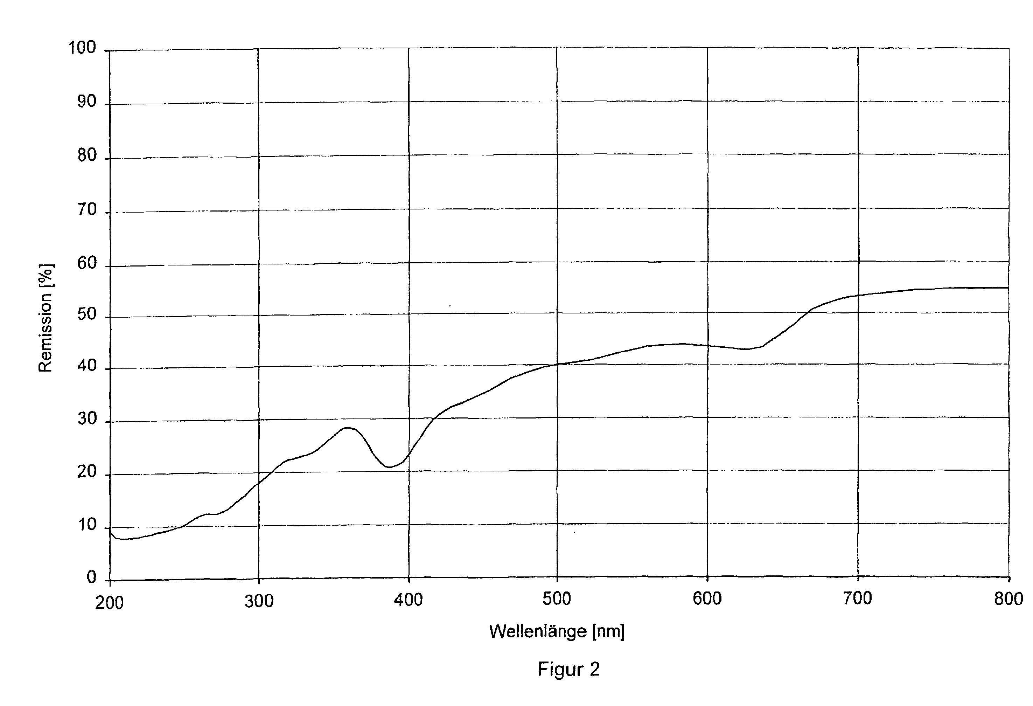

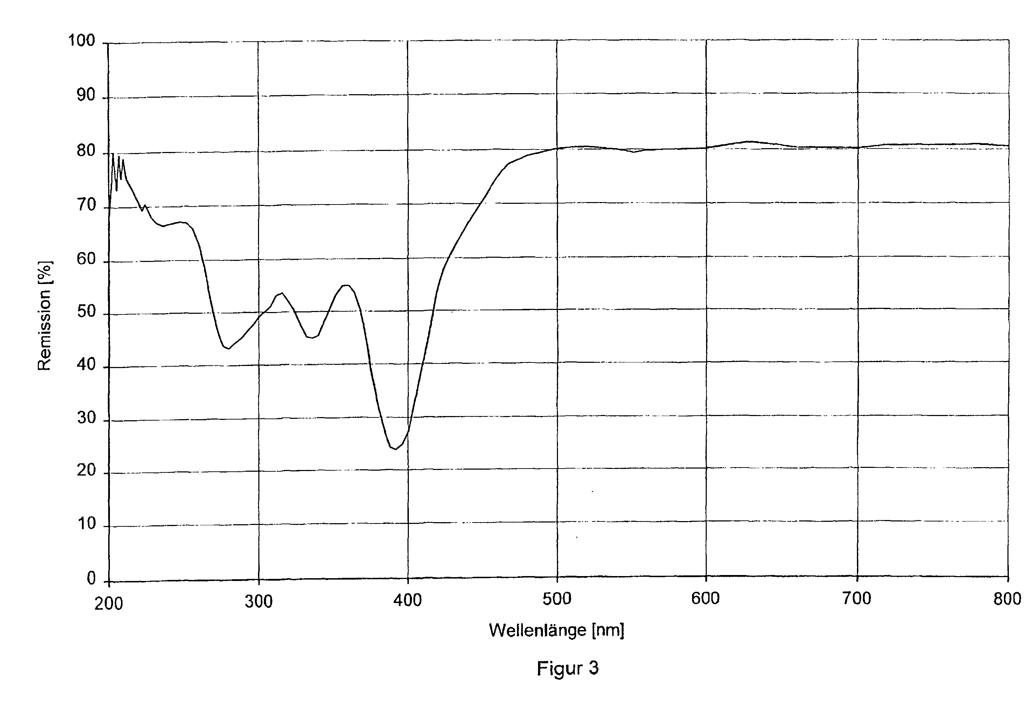

Die in Beispiel 1 und Vergleichsbeispiel 1 erhaltenen Katalysatorkörper unterschieden sich nach der Vorsulfidierung deutlich in ihrer Färbung. Während die erfindungsgemäß hergestellten Katalysatoren eine gelbliche Färbung aufwiesen, besaßen die Vergleichskatalysatoren eine weiße bis graue Einfärbung. Zur Messung der Remission beider Materialien wurde eine gewisse Menge beider Katalysatoren pulverisiert und jeweils zu Tabletten verpreßt. Ihre Remission wurde in einem Perkin-Elmer-Spektrometer gegenüber einem Bariumsulfat-Eichstandard vermessen. Die Meßkurven sind in den Figuren 1 und 2 wiedergegeben. Der erfindungsgemäße Katalysator weist unterhalb von 420 nm eine deutliche Absorptionsbande auf, die zu der beobachteten Gelbfärbung führt. Figur 3 zeigt das Differenzspektrum der beiden Remissionskurven der Figuren 1 und 2. Das Differenzspektrum der beiden macht die Unterschiede zwischen beiden Katalysatoren besonders deutlich. Es treten hierbei drei Absorptionsbanden im Bereich zwischen 270 und 420 nm auf.

### Vergleichsbeispiel 2

Es wurde wie in Vergleichsbeispiel 1 verfahren, jedoch wurde die gesamte Menge Kaliumwolframat in einem Schritt durch Porenvolumenimprägnierung auf die Katalysatorpartikel aufgebracht. Hierzu wurden 240 g Kaliumwolframat (16 Gew.-% der eingesetzten Menge von 1,5 Kg Aluminiumoxid) in 750 ml auf 95°C erwärmtem Wasser gelöst und über die in einem Dragierkessel umgewälzten Katalysatorpartikel verteilt.

### Vergleichsbeispiel 3

Es wurde wie in Vergleichsbeispiel 2 verfahren, die Porenvolumenimprägnierung wurde jedoch in zwei Stufen mit einer Zwischentrocknung der Katalysatorpartikel durchgeführt. Für jede Imprägnierstufe wurden 120 g Kaliumwolframat in 750 cm³ Wasser gelöst.

Die in den vorstehenden Beispielen erhaltenen Katalysatorkörper wurden vor dem Einsatz zur Herstellung von Methylmercaptan vorsulfidiert. Hierbei zeigte sich, daß die erfindungsgemäß hergestellten Katalysatoren sich nach der Sulfidierung deutlich durch ihre Färbung von den Vergleichskatalysatoren unterschieden. Während die erfindungsgemäß hergestellten Katalysatoren eine gelbliche Färbung aufwiesen, besaßen die Vergleichskatalysatoren eine weiße bis graue Einfärbung.

### Anwendungsbeispiel

Die Katalysatoren wurden bezüglich ihrer Leistungsdaten bei der Synthese von Methylmercaptan aus Schwefelwasserstoff und Methanol getestet.

Die Synthese wurde in einem Edelstahlrohr von 14 mm Innendurchmesser und einer Länge von 500 mm durchgeführt. Die Katalysatorschüttung von jeweils 32,4 ml wurde beiderseits durch Inertschüttungen aus Glaskugeln im Reaktionsrohr fixiert. Das Reaktionsrohr wurde elektrisch auf die Reaktionstemperatur von etwa 350°C aufgeheizt.

Die Produkte Methylmercaptan, Dimethylsulfid, Dimethylether und nicht umgesetztes Methanol wurden nach Abkühlen des Produktes mit Methanol bei 25°C aus dem Gasstrom ausgewaschen und destillativ aufgearbeitet.

Die Versuchsbedingungen sind der folgenden Aufstellung zu entnehmen.

| | |
|---|---|
| GHSV | 1280 h⁻¹ (bezogen auf Normbedingungen) |
| LHSV | 0,56 h⁻¹ (bezogen auf flüssiges MeOH) |
| Reaktionstemperatur | 357°C |
| Molverh. H₂S/MeOH | 1,5 |
| Druck | 9 bar |

Die am Reaktionsgasgemisch durch on-line Gaschromatographie gewonnenen Meßergebnisse sind der folgenden Tabelle zu entnehmen. Wie aus dieser Tabelle ersichtlich ist, führt der erfindungsgemäße Katalysator von Beispiel 1 bei gleichem Methanolumsatz zu einer höheren Selektivität bei der Methylmercaptan Ausbeute von etwa 2 % gegenüber dem Vergleichskatalysator von Vergleichsbeispiel 2. Dies führt bei großtechnischer Realisierung der Synthese von Methylmercaptan zu erheblichen Kosteneinsparungen bei der Abtrennung des Reaktionsproduktes. Diese Ergebnisse wurden bei einem relativ geringen Molverhältnis von Schwefelwasserstoff zu Methanol von nur 1,5 sowie bei gegenüber dem Stand der Technik relativ geringen Reaktionstemperaturen von 357°C erzielt.

**Tabelle**

| Versuchsergebnisse | | |
|---|---|---|
| **Katalysator** | **Methanolumsatz** **[%]** | **Selektivität** **[%]** |
| Beispiel 1 | 90 | 91,3 |
| Beispiel 2 | 87,9 | 91,2 |
| Vergleichsbeisp. 1 | 89,9 | 88,7 |
| Vergleichsbeisp. 2 | 90 | 89,2 |
| Vergleichsbeisp. 3 | 89,5 | 89,0 |

Die vorstehenden Ausführungen wurden der Einfachheit halber auf die Probleme bei der Synthese von Methylmercaptan beschränkt. Dem Fachmann ist jedoch klar, daß der erfindungsgemäße Katalysator ebenso für die Synthese allgemeiner Mercaptane durch katalytische Umsetzung von olefinischen Kohlenwasserstoffen mit Schwefelwasserstoff geeignet ist.

## Patentansprüche

1. Katalysator aus Aluminiumoxid für die Synthese von Methylmercaptan enthaltend auf aktivem Aluminiumoxid 5 bis 25 Gew.-% Kaliumwolframat als Promotor,
**dadurch erhältlich,**
daß der Promotor in zwei Portionen auf dem aktiven Aluminiumoxid abgeschieden wird, wobei das Aluminiumoxid zunächst mit der ersten Portion des Promotors durch Imprägnieren in einem Überschuß einer wäßrigen Imprägnierlösung beschichtet und danach bei erhöhter Temperatur getrocknet wird, bevor die zweite Portion mittels der Porenvolumenimprägnierung auf dem Aluminiumoxid aufgebracht und die so erhaltene Katalysatorvorstufe nach erneutem Trocknen bei erhöhter Temperatur abschließend bei Temperaturen zwischen 200 und 600°C calciniert wird.

2. Katalysator nach Anspruch 1,
**dadurch gekennzeichnet,**
**daß** die Menge der ersten Portion des Promotors ein Drittel bis zwei Drittel der Gesamtmenge des Promotors umfaßt.

3. Verfahren zur Herstellung eines Katalysators für die Synthese von Methylmercaptan enthaltend auf aktivem Aluminiumoxid 5 bis 25 Gew.-% Kaliumwolframat als Promotor,
**dadurch gekennzeichnet,**
**daß** der Promotor in zwei Portionen auf dem aktiven Aluminiumoxid abgeschieden wird, wobei das Aluminiumoxid zunächst mit der ersten Portion des Promotors durch Imprägnieren in einem Überschuß einer wäßrigen Lösung beschichtet und danach bei erhöhter Temperatur getrocknet wird, bevor die zweite Portion mittels der Porenvolumenimprägnierung auf dem Aluminiumoxid aufgebracht und die so erhaltene Katalysatorvorstufe nach erneutem Trocknen bei erhöhter Temperatur abschließend bei Temperaturen zwischen 200 und 600°C calciniert wird.

4. Verfahren nach Anspruch 3,
**dadurch gekennzeichnet,**
**daß** die Menge der ersten Portion des Promotors ein Drittel bis zwei Drittel der Gesamtmenge des Promotors umfaßt.

5. Verfahren nach Anspruch 4,
**dadurch gekennzeichnet,**
**daß** das aktive Aluminiumoxid vor der Aufbringung des Promotors für die Dauer von 1 bis 10 Stunden bei Temperaturen zwischen 400 und 600°C calciniert wird.

6. Verwendung des Katalysators nach den Ansprüchen 1 bis 2 für die Synthese von Alkylmercaptanen aus Alkanolen und Schwefelwasserstoff.

7. Verwendung des Katalysators nach den Ansprüchen 1 bis 2 für die Synthese von Methylmercaptan aus Methanol und Schwefelwasserstoff.

## Claims

1. A catalyst consisting of aluminium oxide for the synthesis of methyl mercaptan containing 5 to 25 wt.% of potassium tungstate as promoter on active aluminium oxide,
obtainable in that
the promoter is deposited in two portions on the active aluminium oxide, wherein the aluminium oxide is initially coated with the first portion of promoter by impregnation in an excess of an aqueous impregnation solution and then dried at elevated temperature, before the second portion is applied to the aluminium oxide by pore volume impregnation and the catalyst precursor obtained in this way, after renewed drying at elevated temperature, is then calcined at temperatures between 200 and 600°C.

2. A catalyst according to claim 1,
**characterised in that**
the amount in the first portion of promoter is one third to two thirds the total amount of promoter.

3. A process for preparing a catalyst for the synthesis of methyl mercaptan containing 5 to 25 wt.% of potassium tungstate as promoter on active aluminium oxide,
**characterised in that**
the promoter is deposited on the active aluminium oxide in two portions, wherein the aluminium oxide is initially coated with the first portion of promoter by impregnation in an excess of an aqueous solution and then dried at elevated temperature, before the second portion is applied to the aluminium oxide by pore volume impregnation and the catalyst precursor obtained in this way, after renewed drying at elevated temperature, is then calcined at temperatures between 200 and 600°C.

4. A process according to claim 3,
**characterised in that**
the amount in the first portion of promoter is one third to two thirds the total amount of promoter.

5. A process according to claim 4,
**characterised in that**
the active aluminium oxide is calcined for a period of 1 to 10 hours at temperatures between 400 and 600°C before application of the promoter.

6. Use of the catalyst according to claims 1 to 2 for the synthesis of alkyl mercaptans from alkanols and hydrogen sulfide.

7. Use of the catalyst according to claims 1 to 2 for the synthesis of methyl mercaptan from methanol and hydrogen sulfide.

## Revendications

1. Catalyseur en oxyde d'aluminium pour la synthèse de méthylmercaptan contenant 5 à 25 % en poids de tungstate de potassium comme promoteur sur de l'oxyde d'aluminium actif, pouvant être obtenu en ce que le promoteur est déposé en deux portions sur l'oxyde d'aluminium, l'oxyde d'aluminium étant d'abord revêtu par la première portion du promoteur par imprégnation dans un excès d'une solution aqueuse d'imprégnation et étant ensuite séché à température élevée, avant l'application de la deuxième portion sur l'oxyde d'aluminium au moyen de l'imprégnation du volume de pores et en ce que le précurseur de catalyseur ainsi obtenu, après un nouveau séchage à température élevée, est enfin calciné à des températures entre 200 et 600°C.

2. Catalyseur selon la revendication 1, **caractérisé en ce que** la quantité de la première portion du promoteur comprend un tiers à deux tiers de la quantité totale du promoteur.

3. Procédé pour la préparation d'un catalyseur pour la synthèse de méthylmercaptan contenant 5 à 25 % en poids de tungstate de potassium comme promoteur sur de l'oxyde d'aluminium actif, **caractérisé en ce que** le promoteur est déposé en deux portions sur l'oxyde d'aluminium, l'oxyde d'aluminium étant d'abord revêtu par la première portion du promoteur par imprégnation dans un excès d'une solution aqueuse d'imprégnation et étant ensuite séché à température élevée, avant l'application de la deuxième portion sur l'oxyde d'aluminium au moyen de l'imprégnation du volume de pores et **en ce que** le précurseur de catalyseur ainsi obtenu, après un nouveau séchage à température élevée, est enfin calciné à des températures entre 200 et 600°C.

4. Procédé selon la revendication 3, **caractérisé en ce que** la quantité de la première portion du promoteur comprend un tiers à deux tiers de la quantité totale du promoteur.

5. Procédé selon la revendication 4, **caractérisé en ce que** l'oxyde d'aluminium, avant l'application du promoteur, est calciné pendant une durée de 1 à 10 heures à des températures entre 400 et 600°C.

6. Utilisation du catalyseur selon les revendications 1 à 2 pour la synthèse d'alkylmercaptans à partir d'alcanols et d'acide sulfhydrique.

7. Utilisation du catalyseur selon les revendications 1 à 2 pour la synthèse de méthylmercaptan à partir de méthanol et d'acide sulfhydrique.
